# EUROPÄISCHE PATENTANMELDUNG

(11) **EP 1 818 009 A1**
(43) Veröffentlichungstag der Anmeldung: **15.08.2007**
(21) Anmeldenummer: 06002793.5
(22) Anmeldetag: 11.02.2006
(51) Int. Cl.: A61B 5/00

(54) **Analysehandgerät mit Auswahl von Einstellmodi**

(71) Anmelder: Roche Diagnostics GmbH, 68305 Mannheim (DE); F.HOFFMANN-LA ROCHE AG, 4070 Basel (CH)
(72) Erfinder: Werner, Karl, 69168 Wiesloch (DE); Albrecht, Gertrud, 68239 Mannheim (DE); Supka, Rudolf, Dr., 64646 Heppenheim (DE)
(74) Vertreter: Mommer, Niels

(57) **Zusammenfassung**

Die Erfindung betrifft ein Analysehandgerät zur Untersuchung einer Probe einer menschlichen oder tierischen Körperflüssigkeit, umfassend eine Messeinheit zum Durchführen einer Messung an einer Probe, einen Mikroprozessor zum Steuern der Messeinheit, ein Display (4) zum Anzeigen von Messergebnissen, eine Signaleinrichtung, die im Betrieb zu festgelegten Zeiten ein Erinnerungssignal erzeugt, um an das Durchführen einer Messung zu erinnern, und mindestens ein Bedienungselement, mit dem ein Benutzer Daten und/oder Steuerungsbefehle eingeben kann. wobei das Analysehandgerät derart eingerichtet ist, dass es durch Betätigen des mindestens einen Bedienungselementes (1) in einen Auswahlmodus versetzbar ist, in dem das Gerät mittels des oder der Bedienungselemente (1, 2, 3) in einen von mehreren Einstellmodi (F1, F2, F3, F4) versetzt werden kann, in denen ein Benutzer durch Eingabe von Daten und/oder Steuerungsbefehlen Geräteinstellungen ändern kann, wobei in dem Auswahlmodus jeweils einer der Einstellmodi (F1, F2, F3, F4) ausgewählt ist, so dass das Gerät mittels des oder der Bedienungselemente in den ausgewählten Einstellmodus (F1, F2, F3, F4) versetzbar ist, und wobei in dem Auswahlmodus durch Betätigen des oder der Bedienungselemente (2, 3) einstellbar ist, welcher Einstellmodus (F1, F2, F3, F4) ausgewählt ist.

## Beschreibung

Die Erfindung betrifft ein Analysehandgerät zur Untersuchung einer Probe einer menschlichen oder tierischen Körperflüssigkeit, umfassend eine Messeinheit zum Durchführen einer Messung an einer Probe, einen Mikroprozessor zum Steuern der Messeinheit, ein Display zum Anzeigen von Messergebnissen, und mindestens ein Bedienungselement, mit dem ein Benutzer Daten und/oder Steuerungsbefehle eingeben kann.

Derartige Analysehandgeräte sind handelsüblich erhältlich und werden insbesondere von Diabetikern zur Bestimmung des Blutglucosegehalts verwendet. Diabetiker müssen ihren Blutglucosegehalt mehrmals täglich bestimmen und sind deshalb gezwungen, ein geeignetes Analysehandgerät ständig mit sich zu führen. Daraus ergibt sich die Anforderung, dass Analysehandgeräte zur Bestimmung des Blutglucosegehalts möglichst klein und kompakt sein müssen.

Die Funktion moderner Analysehandgeräte ist nicht auf Messungen zur Untersuchung von Proben beschränkt. Moderne Analysehandgeräte können Messergebnisse mit Schwellenwerten vergleichen und einen Benutzer beispielsweise auf zu hohe oder zu tiefe Blutglucosekonzentrationswerte durch akustische Warnsignale aufmerksam machen oder zu einstellbaren Tageszeiten an das Durchführen einer Messung erinnern. Jede derartige Zusatzfunktion trägt jedoch dazu bei, den Umgang mit dem Analysehandgerät zu verkomplizieren und dessen korrekte Bedienung zu erschweren. Beispielsweise muss bei Geräten mit einer Erinnerungsfunktion eine interne Uhr durch Eingabe einer aktuellen Uhrzeit gestellt werden. Anschließend müssen Erinnerungszeiten programmiert werden, damit ein Benutzer an das Durchführen einer Messung erinnert werden kann. In entsprechender Weise müssen bei Geräten mit einer Warnfunktion Schwellenwerte eingestellt werden, damit ein Benutzer bei medizinisch bedenklichen Blutglucosekonzentrationen durch ein Warnsignal auf gesundheitliche Gefahren aufmerksam gemacht werden kann.

Da bei kleinen und kompakten Analysehandgeräten Anzahl und Größe der Bedienungselemente aus Platzgründen sehr beschränkt sind, ist das Eingeben von Daten und Steuerungsbefehlen zum Einstellen verschiedener Gerätefunktionen für viele Benutzer mühsam und zeitaufwändig. Dies gilt insbesondere für Personen, deren manuelle Geschicklichkeit durch Krankheit oder Alter eingeschränkt ist.

Aufgabe der Erfindung ist es deshalb, einen Weg aufzuzeigen, wie für einen Benutzer das Einstellen eines Analysehandgeräts der eingangs genannten Art gemäß seinem persönlichen Benutzerprofil oder das Ändern einer Geräteeinstellung möglichst einfach gestaltet werden kann.

Diese Aufgabe wird erfindungsgemäß gelöst durch ein Analysehandgerät zur Untersuchung einer Probe einer menschlichen oder tierischen Körperflüssigkeit, umfassend eine Messeinheit zum Durchführen einer Messung an einer Probe, einen Mikroprozessor zum Steuern der Messeinheit, ein Display zum Anzeigen von Messergebnissen, und mindestens ein Bedienungselement, mit dem ein Benutzer Daten und/oder Steuerungsbefehle eingeben kann, wobei das Analysehandgerät derart eingerichtet ist, dass es durch Betätigen des mindestens einen Bedienungselementes in einen Auswahlmodus versetzbar ist, in dem das Gerät mittels des oder der Bedienungselemente in einen von mehreren Einstellmodi versetzt werden kann, in denen ein Benutzer durch Eingabe von Daten und/oder Steuerungsbefehlen Geräteinstellungen ändern kann, wobei in dem Auswahlmodus jeweils einer der Einstellmodi ausgewählt ist, so dass das Gerät mittels des oder der Bedienungselemente in den ausgewählten Einstellmodus versetzbar ist, und wobei in dem Auswahlmodus durch Betätigen des oder der Bedienungselemente einstellbar ist, welcher Einstellmodus ausgewählt ist.

Zum Ändern einer Geräteeinstellung ist es bei dem erfindungsgemäßen Analysehandgerät nicht mehr erforderlich, der Reihe nach verschiedene Geräteeinstellungen zu anderen Gerätefunktionen durchzugehen, die im Regelfall gar nicht geändert werden müssen. Bei einem erfindungsgemäßen Analysehandgerät ist zum Ändern der verschiedenen Geräteeinstellungen jeweils ein spezieller Einstellmodus vorgesehen, der gezielt aktiviert werden kann, um die betreffenden Geräteeinstellungen zu ändern und die entsprechende Gerätefunktionen an ein persönliches Benutzerprofil anzupassen. So können beispielsweise Zeit, Schwellenwerte oder Erinnerungszeiten, zu denen ein Benutzer durch ein Signal an die Durchführung einer Messung erinnert werden soll, unabhängig voneinander eingestellt werden.

Zum Ändern einer Geräteeinstellung wird das Gerät zunächst in den Auswahlmodus versetzt. Aus dem Auswahlmodus kann es durch Betätigen eines Bedienungselements in einen ausgewählten Einstellmodus versetzt werden. Um die Bedienung zu vereinfachen, ist es vorteilhaft, den einzelnen Einstellmodi Symbole (z.B. F1, F2, F3 etc.) zuzuordnen, die auf dem Display angezeigt werden können. Bevorzugt wird in dem Auswahlmodus jeweils einer der Einstellmodi als ausgewählter Einstellmodus angezeigt, beispielsweise indem auf dem Display das den betreffenden Einstellmodus repräsentierendes Symbol (z.B. F1) angezeigt oder hervorgehoben wird. Will der Benutzer das Gerät in den ausgewählten Einstellmodus versetzten, kann die vorgenommene Auswahl durch Betätigen eines Bedienungselements bestätigt werden, so dass das Gerät in den ausgewählten Einstellmodus versetzt wird, also der ausgewählte Einstellmodus aktiviert wird. Wenn sich das Gerät in dem Auswahlmodus befindet und der Benutzer einen anderen als den ausgewählten Einstellmodus nutzen möchte, kann er durch Betätigen eines Bedienungselements ändern, welcher Einstellmodus ausgewählt ist. Ist schließlich der gewünschte Einstellmodus ausgewählt, kann der Benutzer das Gerät in diesen Einstellmodus versetzen.

Weitere Einzelheiten und Vorteile der Erfindung werden anhand eines Ausführungsbeispiels unter Bezugnahme auf die beigefügten Zeichnungen erläutert. Die darin dargestellten Besonderheiten können einzeln oder in Kombination verwendet werden, um bevorzugte Ausgestaltungen der Erfindung zu schaffen. Es zeigen:
- Fig. 1: ein Flussdiagramm mit den Schritten zum Ändern einer Geräteeinstellung eines Ausführungsbeispiels eines erfindungsgemäßen Analysehandgeräts; und
- Fig. 2: ein Ausführungsbeispiel eines Displays eines erfindungsgemäßen Analysehandgeräts.

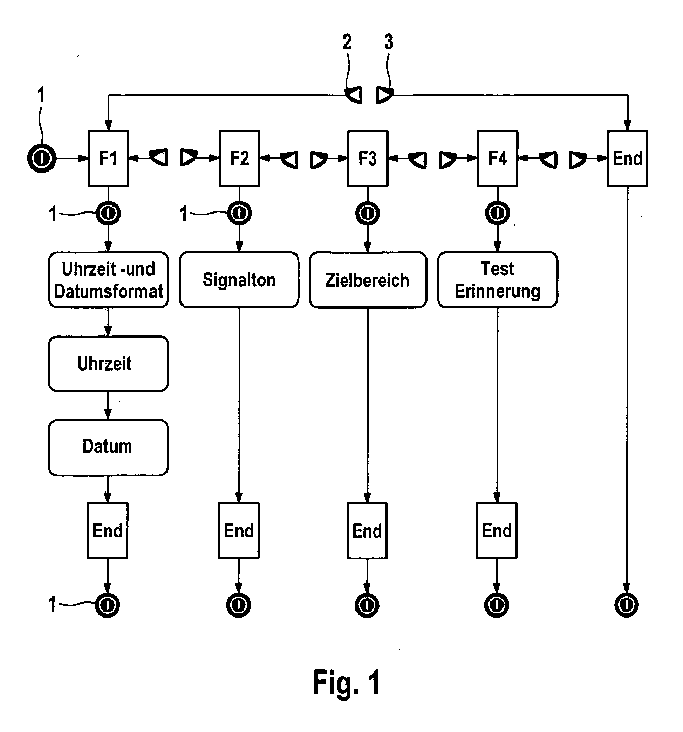

Figur 1 veranschaulicht in einem Flussdiagramm, welche Schritte zum Ändern einer Geräteeinstellung eines erfindungsgemäßen Analysehandgeräts durchzuführen sind. Ein Ausführungsbeispiel eines erfindungsgemäßen Analysehandgeräts ist nicht dargestellt, da es hinsichtlich seiner sichtlichen Bauteile handelsüblichen Analysehandgeräten entspricht.

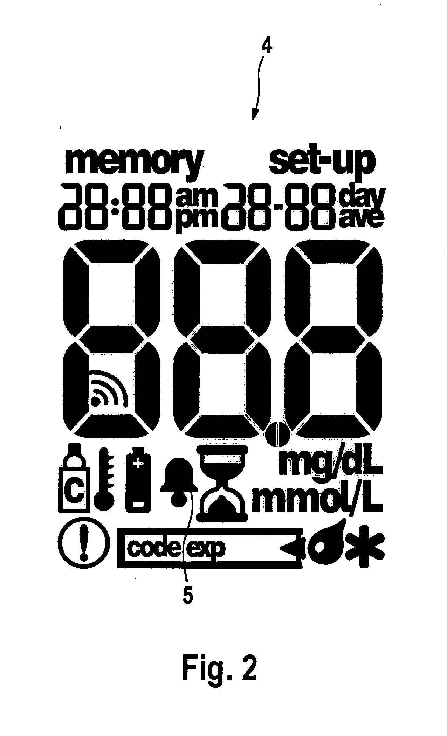

Das Analysehandgerät zur Untersuchung einer Probe einer menschlichen oder tierischen Körperflüssigkeit hat eine Messeinheit zum Durchführen einer Messung an einer Probe, einen Mikroprozessor zum Steuern der Messeinheit, ein in Figur 2 gezeigtes Display 4 zum Anzeigen von Messergebnissen, eine akustische Signaleinrichtung, die im Betrieb zu festgelegten Zeiten ein akustisches Erinnerungssignal erzeugt, um an das Durchführen einer Messung zu erinnern, und drei Bedienungselemente 1, 2, 3, die in dem in Figur 1 dargestellten Flussdiagramm an verschiedenen Stellen in Verbindung mit Pfeilen dargestellt sind. Betätigen des betreffenden Bedienungselements 1, 2, 3 führt jeweils in Pfeilrichtung zu der nächsten Position des Flussdiagramms. Bei den Bedienungselementen 1, 2, 3 handelt es sich bevorzugt um Tasten. Bevorzugt sind nicht mehr als fünf Bedienungselemente 1, 2, 3, besonders bevorzugt nicht mehr als drei Bedienungselemente 1, 2, 3 vorhanden.

Wird das Bedienungselement 1 betätigt, indem es mindestens drei Sekunden lang gedrückt wird, geht das Analysehandgerät in einen Auswahlmodus über, in dem das Gerät mittels der Bedienungselemente 1, 2, 3 in einen von mehreren Einstellmodi F1, F2, F3, F4 versetzt werden kann, in denen ein Benutzer durch Eingabe von Daten und/oder Steuerungsbefehlen Geräteeinstellungen ändern kann. Befindet sich das Gerät in dem Auswahlmodus ist jeweils einer der Einstellmodi F1, F2, F3, F4 ausgewählt.

Bei dem dargestellten Ausführungsbeispiel ist nach dem Aktivieren des Auswahlmodus zunächst automatisch stets der Einstellmodus F1 ausgewählt. Durch Betätigen des Bedienungselements 1 geht das Gerät in den aktuell ausgewählten Einstellmodus über. Gemäß dem in Figur 1 dargestellten Flussdiagramm geht das Analysehandgerät durch längeres Drücken des Bedienungselements 1 also in den Auswahlmodus über, wobei automatisch zunächst der Einstellmodus F1 ausgewählt ist. Durch erneutes Betätigen des Bedienungselements 1 wird das Gerät in den ausgewählten Einstellmodus (z.B. F1) versetzt. In dem Einstellmodus F1 kann mittels der Bedienungselemente 1, 2, 3 in üblicher Weise eine Uhrzeit und ein Datum eingegeben werden, um eine in das Gerät integrierte Uhr zu stellen und eine Datumsanzeige zu ermöglichen. Der erste Einstellmodus F1 wird deshalb als Zeit-Einstellmodus bezeichnet. Ist die Zeit- und Datumseinstellung abgeschlossen, wird das Analysehandgerät durch erneutes Betätigen des Bedienungselements 1 in einen Betriebs- oder Ruhemodus versetzt.

Um eine andere Geräteeinstellung zu ändern, lässt sich in dem Auswahlmodus durch Betätigen der Bedienungselemente 2, 3 ändern, welcher Einstellmodus F1, F2, F3, F4 ausgewählt ist. Betätigen des Bedienungselements 3 führt dabei dazu, dass aus einer geräteinternen gespeicherten Liste der Einstellmodi F1, F2, F3, F4 jeweils der nächst höhere Einstellmodus ausgewählt wird. Betätigen des Bedienungselements 2 führt in entsprechender Weise dazu, dass jeweils der vorhergehende Einstellmodus F1, F2, F3, F4 aus der Liste ausgewählt wird.

Ist der erste Einstellmodus F1 der Liste ausgewählt, führt Betätigen des Bedienungselements 2 zum Verlassen des Auswahlmodus, was in Figur 1 durch das Symbol "End" dargestellt ist. Betätigen des Bedienungselements 1 führt dann dazu, dass das Gerät in einen Betriebs- oder Ruhemodus übergeht. In entsprechender Weise führt Betätigen des Bedienungselements 3 zu einem Verlassen des Auswahlmodus, wenn der letzte Einstellmodus F4 der Liste ausgewählt ist.

Der zweite Einstellmodus F2 dient dazu, das Gerät in einen Akustikmodus zu versetzen, in dem Messergebnisse für sehbehinderte Personen durch akustische Signale angezeigt werden können. Der zweite Einstellmodus F2 wird deshalb als Akustik-Einstellmodus bezeichnet. In dem zweiten Einstellmodus F2 kann eine akustische Bedienerführung aktiviert werden, in der akustische Hinweise, insbesondere in Form von Sprache, als Bedienungshilfe ausgegeben werden. Zum Aktivieren der akustischen Bedienerführung wird in dem Einstellmodus F2 der Zustand ON1 eingestellt. Daneben in dem Einstellmodus F2 der Akustikmodus auch vollständig aktiviert werden, so dass auch Messergebnisse akustisch ausgegeben werden, indem der Zustand ON2 eingestellt wird. Ist keine Akustikausgabe gewünscht, kann in dem Einstellmodus F2 der Zustand OFF eingestellt werden, so dass weder eine akustische Ausgabe von Messergebissen noch eine akustische Bedienerführung erfolgt.

Wird der dritte Einstellmodus F3 aktiviert, lassen sich Schwellenwerte einprogrammieren, mit denen der Mikroprozessor mittels der Messeinheit zu ermittelnde Messwerte vergleicht und die Signaleinrichtung betätigt, wenn ein Messwert einen oberen Schwellenwert überschreitet oder einen unteren Schwellenwert unterschreitet, so dass beim Eintreten eines solchen Falls von der Signaleinrichtung ein akustisches Warnsignal erzeugt wird. Der dritte Einstellmodus F3 wird deshalb als Schwellenwert-Einstellmodus bezeichnet.

Befindet sich das Analysehandgerät in dem vierten Einstellmodus F4, können Erinnerungszeiten programmiert werden, zu denen die Signaleinrichtung von dem Mikroprozessor betätigt wird und ein Erinnerungssignal erzeugt, um einen Benutzer an das Durchführen einer Messung zu erinnern. Der vierte Einstellmodus F4 wird deshalb als Wecker-Einstellmodus bezeichnet.

Jedem der Einstellmodi F1, F2, F3, F4 ist ein Symbol zugeordnet, das auf dem Display 4 angezeigt wird, wenn sich das Gerät in dem Auswahlmodus befindet und der betreffende Einstellmodus F1, F2, F3, F4 ausgewählt ist. Als Symbole für die verschiedenen Einstellmodi sind Kombinationen eines Buchstabens mit einer Zahl besonders geeignet, da sich derartige Symbole mit geringem Aufwand auf einem LCD-Display 4 darstellen lassen, insbesondere können derartige Symbole auch auf einem kostengünstigen Segmentdisplay 4 dargestellt werden, wie es in Figur 2 dargestellt ist.

Ein Segmentdisplay 4 mit einer Fixsegmentanzeige hat zwar weniger graphische Möglichkeiten als eine Dot-Matrix, ist jedoch auch wesentlich kostengünstiger. Bevorzugt ist das Segmentdisplay 4 dafür eingerichtet, neben Messergebnissen und Symbolen der Einstellmodi F1, F2, F3, F4 auch einfache graphische Symbole als Bedienungshilfe anzuzeigen, beispielsweise eine Glocke 5 als Symbol dafür, dass die Erinnerungsfunktion aktiviert und eine Erinnerungszeit eingestellt ist.

Bevorzugt ist das Analysehandgerät netzunabhängig betreibbar und verfügt zu diesem Zweck über eine netzunabhängige Energieversorgung, beispielsweise Batterien oder Solarzellen.

## Patentansprüche

**1.** Analysehandgerät zur Untersuchung einer Probe einer menschlichen oder tierischen Körperflüssigkeit, umfassend:
- eine Messeinheit zum Durchführen einer Messung an einer Probe,
- einen Mikroprozessor zum Steuern der Messeinheit,
- ein Display (4) zum Anzeigen von Messergebnissen, und
- mindestens ein Bedienungselement (1, 2, 3), mit dem ein Benutzer Daten und/oder Steuerungsbefehle eingeben kann,
wobei das Analysehandgerät derart eingerichtet ist, dass es durch Betätigen des mindestens einen Bedienungselementes (1) in einen Auswahlmodus versetzbar ist, in dem das Gerät mittels des oder der Bedienungselemente (1, 2, 3) in einen von mehreren Einstellmodi (F1, F2, F3, F4) versetzt werden kann, in denen ein Benutzer durch Eingabe von Daten und/oder Steuerungsbefehlen Geräteinstellungen ändern kann,
wobei in dem Auswahlmodus jeweils einer der Einstellmodi (F1, F2, F3, F4) ausgewählt ist, so dass das Gerät mittels des mindestens einen Bedienungselementes in den ausgewählten Einstellmodus (F1, F2, F3, F4) versetzbar ist, und
wobei in dem Auswahlmodus durch Betätigen des oder der Bedienungselemente (2, 3) einstellbar ist, welcher Einstellmodus (F1, F2, F3, F4) ausgewählt ist.

**2.** Analysehandgerät nach Anspruch 1, **dadurch gekennzeichnet, dass** einer der Einstellmodi (F1) ein Zeit-Einstellmodus ist, in dem eine Uhrzeit eingegeben werden kann, um eine in das Gerät integrierte Uhr zu stellen.

**3.** Analysehandgerät nach einem der vorhergehenden Ansprüche, **gekennzeichnet durch** eine Signaleinrichtung, die zu einstellbaren Zeiten ein Erinnerungssignal erzeugt, um an das Durchführen einer Messung zu erinnern.

**4.** Analysehandgerät nach Anspruch 3, **dadurch gekennzeichnet, dass** einer der Einstellmodi (F4) ein Wecker-Einstellmodus ist, in dem Erinnerungszeiten programmiert werden können, zu denen die Signaleinrichtung von dem Mikroprozessor betätigt wird und ein Erinnerungssignal erzeugt, um einen Benutzer an das Durchführen einer Messung zu erinnern.

**4.** Analysehandgerät nach einem der vorhergehenden Ansprüche, **dadurch gekennzeichnet, dass** einer der Einstellmodi (F3) ein Schwellenwert-Einstellmodus ist, in dem Schwellenwerte einprogrammiert werden können, mit denen der Mikroprozessor mittels der Messeinheit zu ermittelnde Messwerte vergleicht und die Signaleinrichtung betätigt, wenn ein Messwert einen oberen Schwellenwert überschreitet oder einen unteren Schwellenwert unterschreitet, so dass bei Eintreten eines solchen Falls von der Signaleinrichtung ein Warnsignal erzeugt wird.

**5.** Analysehandgerät nach einem der vorhergehenden Ansprüche, **dadurch gekennzeichnet, dass** einer der Einstellmodi (F2) ein Akustik-Einstellmodus ist, in dem ein Akustikmodus aktivierbar ist, in dem Messergebnisse durch akustische Signale angezeigt werden oder eine akustische Bedienungsführung erfolgt.

**6.** Analysehandgerät nach einem der vorhergehenden Ansprüche, **dadurch gekennzeichnet, dass** das Analysehandgerät derart eingerichtet ist, dass es durch Betätigen eines ersten Bedienungselements
(1) in den Auswahlmodus versetzt werden kann, aus dem es durch Betätigen des ersten Bedienungselements (1) oder eines zweiten Bedienungselements (2, 3) in einer der Einstellungsmodi (F1, F2, F3, F4) versetzt werden kann.

**7.** Analysehandgerät nach Anspruch 6, **dadurch gekennzeichnet, dass** es in dem Auswahlmodus durch Betätigen des ersten Bedienungselements (1) in den ausgewählten Einstellmodus (F1, F2, F3, F4) versetzbar ist.

**8.** Analysehandgerät nach Anspruch 6 oder 7, **dadurch gekennzeichnet, dass** es derart ausgebildet ist, dass in dem Auswahlmodus durch Betätigen des zweiten Bedienungselements (2, 3) einstellbar ist, welcher Einstellmodus (F1, F2, F3, F4) ausgewählt ist.

**9.** Analysehandgerät nach einem der vorhergehenden Ansprüche, **dadurch gekennzeichnet, dass** jedem Einstellmodus (F1, F2, F3, F4) ein Symbol zugeordnet ist, das auf dem Display (4) angezeigt wird, wenn sich das Gerät in dem Auswahlmodus befindet und der betreffende Einstellmodus (F1, F2, F3, F4) ausgewählt ist.

**10.** Analysehandgerät nach einem der vorhergehenden Ansprüche, **dadurch gekennzeichnet, dass** das erste und das zweite Bedienungselement (1, 2, 3) Tasten sind.

**11.** Analysehandgerät nach einem der vorhergehenden Ansprüche, **dadurch gekennzeichnet, dass** die Signaleinrichtung eine akustische Signaleinrichtung zum Erzeugen akustischer Signal ist.

**12.** Analysehandgerät nach einem der vorhergehenden Ansprüche, **dadurch gekennzeichnet, dass** es nicht mehr als fünf Bedienungselemente (1, 2, 3), vorzugsweise nicht mehr als drei Bedienungselemente (1, 2, 3), aufweist.

**13.** Analysehandgerät nach einem der vorhergehenden Ansprüche, **dadurch gekennzeichnet, dass** das Display (4) ein Segmentdisplay ist.

**14.** Analysehandgerät nach einem der vorhergehenden Ansprüche, **dadurch gekennzeichnet, dass** es netzunabhängig betreibbar ist.
